# EUROPEAN PATENT SPECIFICATION

(11) **EP 4 205 628 B1**
(45) Date of publication and mention of the grant of the patent: **22.04.2026**
(21) Application number: 20959727.7
(22) Date of filing: 27.10.2020
(51) Int. Cl.: A61B 1/00, A61B 1/045, A61B 3/13, A61B 34/00, A61B 90/00, A61B 90/50, A61F 9/007, G02B 21/00, A61B 34/20, A61B 34/10, G02B 7/00

(54) **SURGERY ASSISTANCE DEVICE**
OPERATIONSUNTERSTÜTZUNGSVORRICHTUNG
DISPOSITIF D'ASSISTANCE CHIRURGICALE

(43) Date of publication of application: 05.07.2023
(73) Proprietor: RIVERFIELD Inc., 1070052 Tokyo (JP)
(72) Inventor: SONODA, Koh-hei, Fukuoka-shi, Fukuoka 819-0395 (JP); NAKAO, Shintaro, Fukuoka-shi, Fukuoka 819-0395 (JP); TADANO, Kotaro, Tokyo 152-8550 (JP); MORIKAWA, Atsushi, Tokyo 160-0017 (JP)
(74) Representative: Adares PartGmbB
(86) International application number: PCT/JP2020/040230
(87) International publication number: WO 2022/091209

(56) References cited:
- WO-A1-2017/169823
- JP-A- 2001 204 738
- JP-A- 2004 105 539
- US-A1- 2019 000 563
- US-A1- 2019 038 364
- US-A1- 2019 105 113
- US-A1- 2020 015 899

## Description

### Technical Field

The present invention relates to a technical field of a surgery assistance device having a function of holding an endoscope.

### Background Art

Vitreous body surgery is known which restores a retina to a normal state by sucking and removing a vitreous body within an eyeball, for example, for treatment of maculopathy, retinal detachment, or the like.

**In** ordinary vitreous body surgery, an operator (surgeon) observes the inside of the eyeball through the pupil of a subject (patient) by a surgical microscope or the like. There is a limit to a range in which the inside of the eyeball can be observed through the pupil. In order to bring the part that cannot be observed into a viewable range, the eyeball needs to be pressed from the outside. Such pressing may cause a pain during the surgery or inflammation after the surgery.

Accordingly, a method of using an endoscope for vitreous body surgery as illustrated in PTL 1 has been proposed.

When the endoscope is inserted into the eyeball of the subject, video of the inside of the eyeball is displayed by display means such as a monitor. The operator can easily observe a part normally unable to be viewed from the pupil by moving the endoscope within the eyeball.

The vitreous body surgery using such an endoscope obviates a need for pressing the eyeball in observing the inside of the eyeball. A burden on the eyeball of the subject can therefore be reduced.
PTL2 describes a system and method for determining the position and orientation of a tool-tip relative to an eye tissue, wherein the system includes an imaging and tracking module coupled with a processor.
PTL3 discloses an image processing device comprising an acquisition unit that acquires a microscopic image obtained by photographing a surgical member inserted to a subject with a surgical microscope, an estimation unit that estimates a relative posture of the surgical member in the subject on the basis of the microscopic image acquired by the acquisition unit, and an output unit that outputs posture information associated with the posture that has been estimated.
PTL4 describes a surgical visualization system comprising a surgical device configured to emit a structured light pattern onto a surface, an image sensor configured to identify a structure embedded below the surface, and a control circuit in signal communication with the image sensor. The control circuit is configured to receive imaging data indicative of the structured light pattern on the surface, generate a three-dimensional digital representation of the surface based on the imaging data, obtain an image of the structure and the surgical device from the image sensor, overlay the image of the structure and the surgical device with the three-dimensional digital representation of the surface on the display screen; and determine a distance from the surgical device to the structure from the image.
PTL5 describes a robot controller including a first input configured to receive images from an imaging device configured to identify a target region in the images.

### Citation List

### Patent Literature

PTL 1: JP 2005-304633 A
PTL 2: US 2019/000563 A1
PTL 3: US 2019/038364 A1
PTL4: US 2020/015899 A1
PTL5: US 2019/105113 A1

### Summary of Invention

The present invention is defined by the features of the independent claim. Embodiments of the invention are defined in the dependent claims.

### Technical Problems

In vitreous body surgery, the surgery is performed by inserting a treatment instrument such as a vitreous body cutter, forceps, or an injector of a perfusate or the like into an eyeball. However, in performing the surgery, the position of an endoscope inserted in the eyeball is not accurately known. Therefore, smooth proceeding of the surgery may be hindered, for example, when the treatment instrument comes into contact with the endoscope during the surgery.

In addition, in inserting the endoscope into the eyeball, when the position of the endoscope in the eyeball cannot be recognized, the endoscope may come into contact with the retina, and damage the retina.

It is accordingly an object of the present invention to make display to enable an operator to recognize positional relation between the eyeball and the endoscope. Solution to Problems

A surgery assistance device according to the present disclosure includes an arm unit having a holder for holding an endoscope, and configured to adjust a position of the endoscope in a state in which the holder holds the endoscope, a measuring unit having fixed relative positional relation to the endoscope held by the holder, and used to measure a distance to a surgery target region of a subject, a position determining section configured to obtain positional relation between the surgery target region of the subject and the endoscope distal end portion on a basis of information from the measuring unit, an image generating section configured to generate a map image indicating the position of the endoscope on a three-dimensional model of the surgery target region by using a determination result of the position determining section, and a display control section configured to perform display control of the map image.

The surgery target region refers to a region of the subject to be operated by an operator. The surgery target region is the eyeball of the subject in vitreous body surgery.

Consequently, the map image is displayed on a monitor. By checking the monitor, the operator can, for example, recognize the position of the endoscope inserted in the eyeball of the subject.

In the surgery assistance device described above, it is considered that the surgery target region is an eyeball, the measuring unit includes an imaging unit and an irradiating unit, and the position determining section determines positional relation between the eyeball of the subject and the endoscope distal end portion on a basis of a captured image of a light spot of light from the irradiating unit, the light spot appearing on the eyeball of the subject, the captured image being captured by the imaging unit.

For example, a distance from the imaging unit to the eyeball can be calculated on the basis of the captured image. The positional relation between the eyeball and the endoscope distal end can be determined on the basis of the distance.

In the surgery assistance device described above, it is considered that the image generating section generates a presenting image including information regarding an insertion length of the endoscope in the eyeball and information regarding a distance from the endoscope distal end portion to a retina of the subject.

Consequently, the numerical value of the insertion length of the endoscope in the eyeball and the numerical value of the distance to the retina of the subject, for example, are displayed on the monitor.

In the surgery assistance device described above, it is considered that the display control section displays the map image and a captured image captured by the endoscope (endoscope captured image) within a same screen.

Because the map image and the endoscope captured image are displayed within the same screen, when the operator performs the surgery while checking the captured image of the inside of the eyeball, the operator can easily recognize the positional relation of the endoscope in the eyeball at the same time.

In the surgery assistance device described above, it is considered that the image generating section updates the map image according to a shift in the position of the endoscope.

Consequently, the map image illustrating the most recent positional relation between the eyeball and the endoscope is displayed on the monitor.

### Advantageous Effect of Invention

According to the present disclosure, the operator can perform surgery while recognizing the position of the endoscope inserted in the surgery target region of the subject.

### Brief Description of Drawings

FIG. 1 is a diagram schematically illustrating an example of a configuration of a surgery system in an embodiment of the present disclosure.
FIG. 2 is a diagram schematically illustrating a sectional configuration of an eyeball of a subject in the present embodiment.
FIG. 3 is a diagram schematically illustrating an example of a configuration of a surgery assistance device in the present embodiment.
FIG. 4 is a diagram schematically illustrating an example of a configuration of an arm distal end section of an endoscope holding device in the present embodiment.
FIG. 5 is a diagram illustrating an example of a display image displayed on a monitor in the present embodiment.
FIG. 6 is a block diagram illustrating an example of a configuration of the surgery assistance device in the present embodiment.
FIG. 7 is a flowchart illustrating an example of processing performed by a computation and control unit of the surgery assistance device in the present embodiment.
FIG. 8 is a diagram illustrating an outline of a procedure for generating an ocular map image in the present embodiment.

### Description of Embodiment

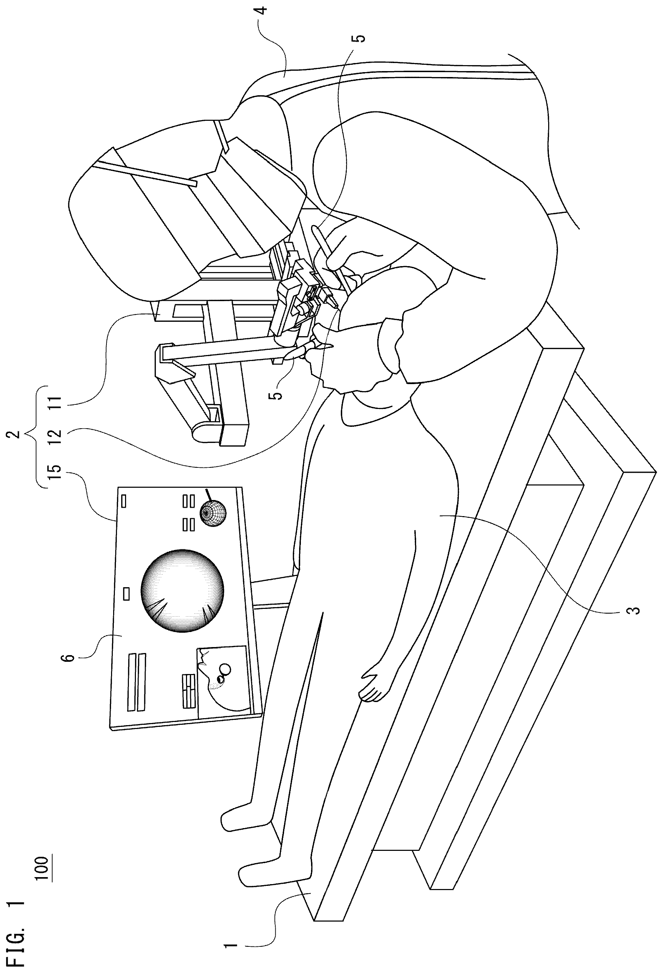

An embodiment of the present disclosure will be described with reference to FIGS. 1 to 8. The drawings extract and illustrate principal parts and peripheral configurations thereof recognized to be necessary for description. In addition, the drawings are schematic, and the dimensions, ratios, and the like of respective structures included in the drawings are mere examples. Hence, various changes can be made according to design or the like without departing from technical ideas of the present disclosure. In addition, configurations described once may be subsequently identified by the same reference numerals, and description thereof may be omitted.

The embodiment will hereinafter be described in the following order.
<1. Configuration of Surgery System>
<2. Functional Configuration of Computation and Control Unit>
<3. Example of Processing of Embodiment>
<4. Summary>

### <1. Configuration of Surgery System>

A configuration of a surgery system 100 in ocular surgery will be described.

FIG. 1 schematically illustrates an example of the configuration of the surgery system 100.

The surgery system 100 includes an operating table 1 and a surgery assistance device 2.

The operating table 1 and the surgery assistance device 2 are installed in an operating room.

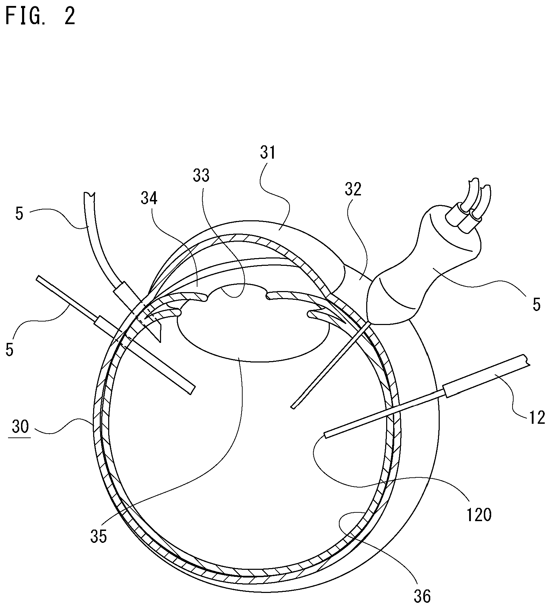

A subject (patient) 3 is laid down on his or her back on the operating table 1. An operator (surgeon) 4 is positioned on the head side of the subject 3, and performs surgery within an eyeball 30 (see FIG. 2) of the subject 3 by using various kinds of treatment instruments 5. Used as the treatment instruments 5 are, for example, a vitreous body cutter, forceps, an injector of a perfusate or the like, and the like.

FIG. 2 schematically illustrates a sectional configuration of the eyeball 30. The surface of the eyeball 30 is covered by a cornea 31 and a conjunctiva 32. An iris 34 in which a pupil 33 is formed is present in the rear of the cornea 31. A crystalline lens 35 is present in the rear of the iris 34. In addition, a retina 36 is present on the whole surface of an ocular fundus within the eyeball 30.

The operator 4, for example, inserts the treatment instruments 5 through the conjunctiva 32, and performs surgery within the eyeball 30.

The surgery assistance device 2 assists in the surgery on the eyeball 30 by the operator 4.

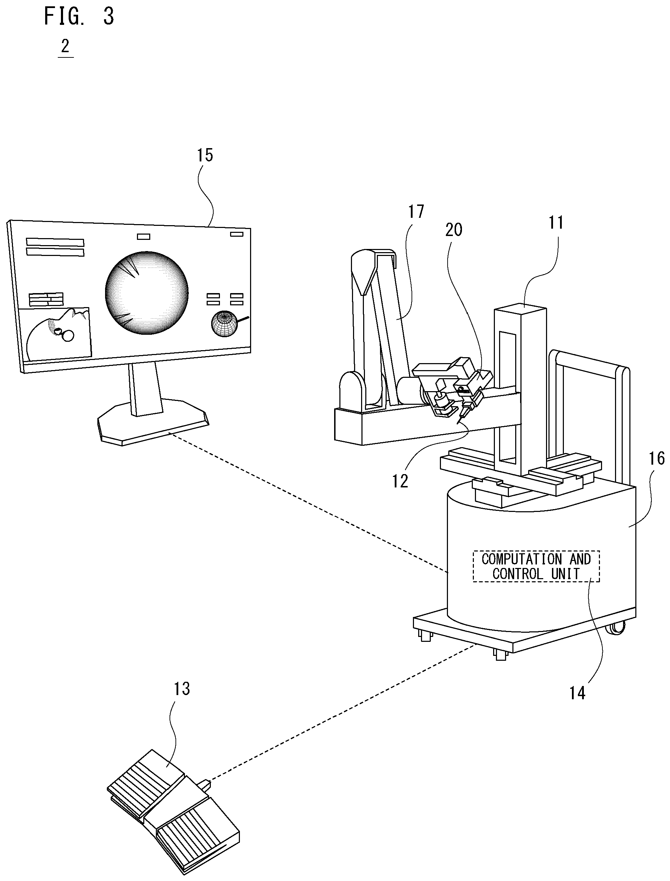

FIG. 3 schematically illustrates an example of a configuration of the surgery assistance device 2.

The surgery assistance device 2 includes an endoscope holding device 11, an endoscope 12, an operating unit 13, a computation and control unit 14, and a monitor 15.

The endoscope holding device 11 includes a base unit 16 and an arm unit 17.

The base unit 16 is mounted on the floor of the operating room or the like. The arm unit 17 is attached to the base unit 16. The arm unit 17 is pivotally supported by the base unit 16 in a rotatable manner.

The arm unit 17 includes one or a plurality of joint sections and rotary sections, and is formed as a mechanism that can move an arm distal end section 20 to a given position.

A configuration of the arm distal end section 20 will be described in the following.

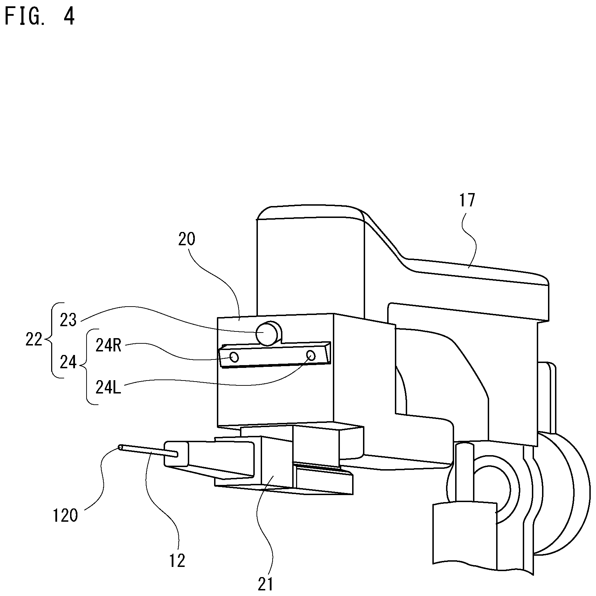

FIG. 4 schematically illustrates an example of the configuration of the arm distal end section 20.

The arm distal end section 20 includes a holder 21 for holding the endoscope 12 and a measuring unit 22 used to measure a distance to the cornea 31 of the subject 3.

The holder 21 is formed as a mechanism that allows the endoscope 12 to be attached to and detached from the holder 21. The endoscope 12 is fixed to the holder 21 by fitting the endoscope 12 into the holder 21. The endoscope 12 can be freely moved to a given position by operating the arm unit 17 in a state in which the endoscope 12 is fixed to the holder 21.

When the holder 21 holds the endoscope 12 inserted in the eyeball 30 of the subject 3, the operator 4 does not need to hold the endoscope 12 with a hand. Hence, the operator 4 can perform surgery on the eyeball 30 with both hands.

The measuring unit 22 includes an irradiating unit 23 and an imaging unit 24.

The irradiating unit 23 includes an LED (Light Emitting Diode), for example. The irradiating unit 23 outputs light that irradiates the eyeball 30 of the subject 3.

The imaging unit 24 includes imaging units 24L and 24R to be able to perform distance measurement by what is called a stereo method. The imaging units 24L and 24R are, for example, arranged at a predetermined interval from each other in the vicinity of an upper portion of the holder 21. Optical axes of the imaging units 24L and 24R are parallel with each other, and respective focal lengths of the imaging units 24L and 24R are the same value. In addition, frame periods thereof are in synchronism with each other, and frame rates thereof coincide with each other.

Captured image signals obtained by respective imaging elements of the imaging units 24L and 24R are each subjected to A/D (Analog/Digital) conversion, and are thereby converted into digital image signals (captured image data) indicating luminance values based on a predetermined gray scale in pixel units.

Distances from the imaging units 24L and 24R to the cornea 31 of the subject 3 can be measured on the basis of captured image signals from the respective imaging elements of the imaging units 24L and 24R, the captured image signals being obtained in a state in which the irradiating unit 23 irradiates the eyeball 30. Details of a method of measuring the distances to the cornea 31 of the subject 3 and a method of utilizing the measured distances will be described later.

In the measuring unit 22, relative positional relation between the irradiating unit 23 and the imaging units 24L and 24R is fixed. In addition, relative positional relation between the imaging units 24L and 24R and the above-described holder 21 is fixed. Hence, relative positional relation of the irradiating unit 23 and the imaging units 24L and 24R to the endoscope 12 is fixed by fixing the endoscope 12 to the holder 21.

Returning to FIG. 3, the endoscope 12 of the surgery assistance device 2 is inserted into the eyeball 30 in a state in which the endoscope 12 is fixed to the holder 21 (see FIG. 2). A state within the eyeball 30 is obtained by the inserted endoscope 12. Captured image signals obtained by the imaging element of the endoscope 12 are each subjected to A/D conversion, and are thereby converted into digital image signals (captured image data) indicating luminance values based on a predetermined gray scale in pixel units.

A captured image based on the captured image data from the endoscope 12 is displayed on the liquid crystal of the monitor 15.

The operating unit 13 comprehensively represents operating equipment used to perform an operation of the arm unit 17, a rotational operation of the captured image, which is displayed on the monitor 15, based on imaging by the endoscope 12, and the like. The operating unit 13 may be a foot pedal, or may be a manually operated remote operating device (remote controller) or the like. FIG. 3 illustrates a foot pedal as an example. However, as described above, the operating unit 13 is not limited to this.

The computation and control unit 14 performs various kinds of processing necessary to implement the present embodiment, such as control of operation of the arm unit 17, processing of generating various kinds of images to be displayed on the monitor 15, and processing of controlling display on the monitor 15.

The computation and control unit 14, for example, includes a microcomputer including a CPU (Central Processing Unit), a ROM (Read Only Memory), a RAM (Random Access Memory), and the like. The computation and control unit 14 is implemented by one or a plurality of microcomputers.

The computation and control unit 14 is, for example, included in the base unit 16 of the endoscope holding device 11. Incidentally, the computation and control unit 14 may be included in another external apparatus.

The monitor 15 displays a display image 6 on the liquid crystal under display control from the computation and control unit 14.

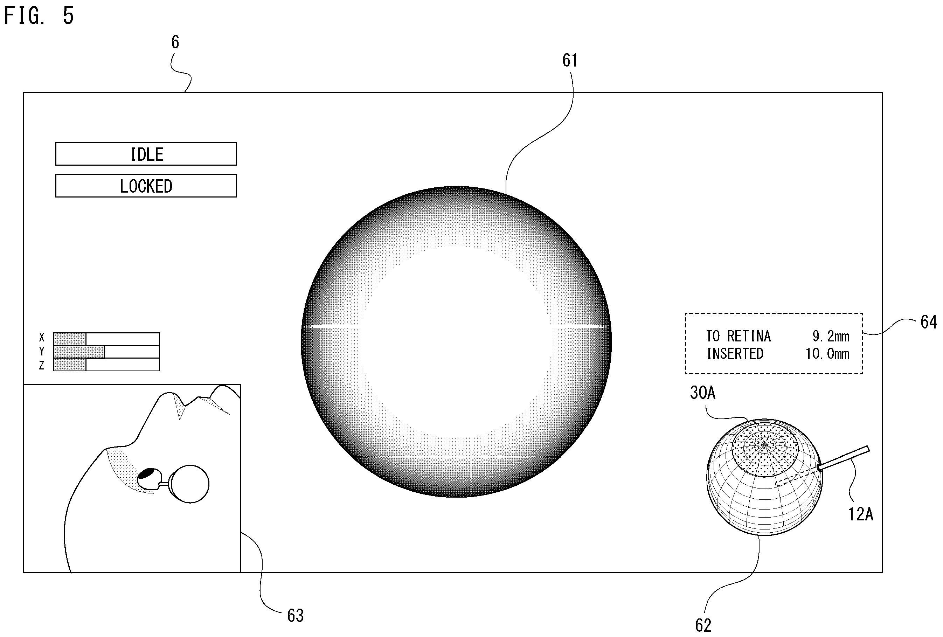

FIG. 5 illustrates an example of the display image 6 displayed on the monitor 15.

The display image 6, for example, including an endoscope captured image 61, an ocular map image 62, an endoscope viewpoint map image 63, an insertion length presenting image 64, and the like is displayed on the monitor 15. The display image 6 also includes images related to various kinds of information as required.

The endoscope captured image 61 is the captured image based on the captured image data from the endoscope 12. A state inside the eyeball 30, which is obtained by the endoscope 12, for example, is displayed as the endoscope captured image 61.

The ocular map image 62 illustrates positional relation between the eyeball 30 and the endoscope 12.

The ocular map image 62 displays the eyeball 30 by a three-dimensional ocular model 30A. In addition, the position of the endoscope 12 with respect to the eyeball 30 is displayed by an endoscope model 12A.

The endoscope viewpoint map image 63 displays a three-dimensional model image of the subject 3 from the viewpoint of the endoscope 12.

The insertion length presenting image 64 displays the numerical value of an insertion length of the endoscope 12 with respect to the eyeball 30 and the numerical value of a distance from an endoscope distal end portion 120 to the retina 36.

The operator 4 performs surgery on the eyeball 30 while checking the display image 6 displayed on the monitor 15.

### <2. Functional Configuration of Computation and Control Unit>

A functional configuration of the computation and control unit 14 in the surgery assistance device 2 will be described.

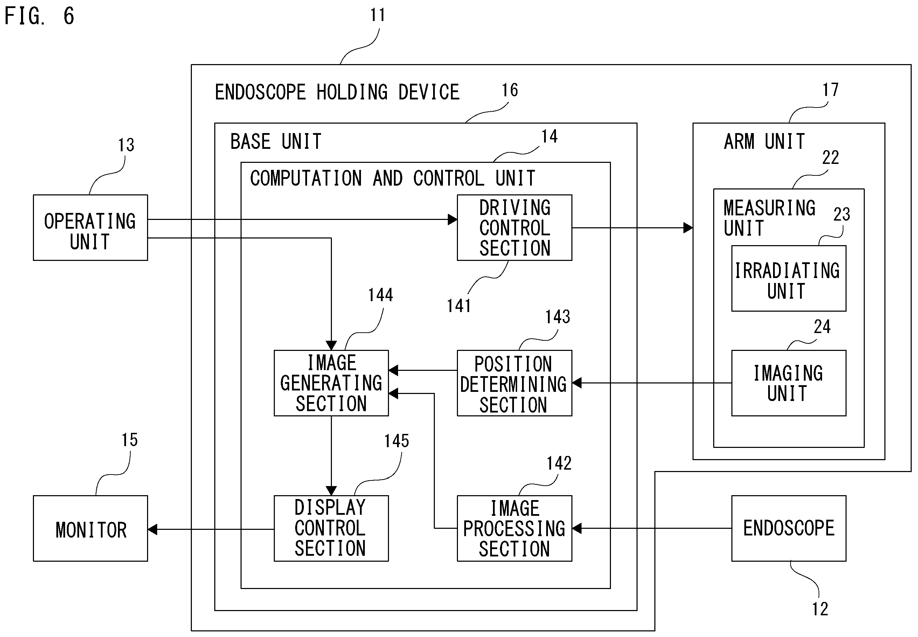

FIG. 6 illustrates, as a block diagram, an example of a configuration of the surgery assistance device 2.

The computation and control unit 14 includes a driving control section 141, an image processing section 142, a position determining section 143, an image generating section 144, and a display control section 145.

The driving control section 141 performs operation control on the joint section(s) and the rotary section(s) of the arm unit 17 of the endoscope holding device 11 on the basis of an operation signal input from the operating unit 13, for example. The driving control section 141 can move the position of the endoscope 12 fixed to the holder 21 of the arm distal end section 20 by performing operation control on the arm unit 17.

In addition, the driving control section 141 performs output control on the irradiating unit 23 and imaging control on the imaging unit 24.

The image processing section 142 subjects the image signal based on imaging by the endoscope 12 to various kinds of signal processing such as luminance signal processing, color processing, resolution conversion processing, and codec processing. The image processing section 142 outputs the image signal resulting from the various kinds of signal processing to the image generating section 144.

The position determining section 143 obtains distance information from the imaging unit 24 to the cornea 31 on the basis of the captured image signals of the eyeball 30 from the respective imaging elements of the imaging units 24L and 24R, the captured image signals being input from the imaging unit 24.

In addition, the position determining section 143 computes relative positional relation between the eyeball 30 and the endoscope distal end portion 120 on the basis of the distance information. Details of a method of computing the relative positional relation will be described later.

The position determining section 143 outputs a determination result (relative positional relation between the eyeball 30 and the endoscope distal end portion 120) to the image generating section 144.

The image generating section 144 generates the display image 6 as illustrated in FIG. 5 by using various kinds of input information from the image processing section 142, the position determining section 143, the operating unit 13, and the like. Details of a method for generating various kinds of images constituting the display image 6 will be described later.

The image generating section 144 outputs an image signal of the generated display image 6 to the display control section 145.

The display control section 145 performs control that displays the display image 6 on the monitor 15 on the basis of the image signal input from the image generating section 144.

### <3. Example of Processing of Embodiment>

Description will be made of processing performed by the computation and control unit 14 of the surgery assistance device 2 in order to implement the present embodiment.

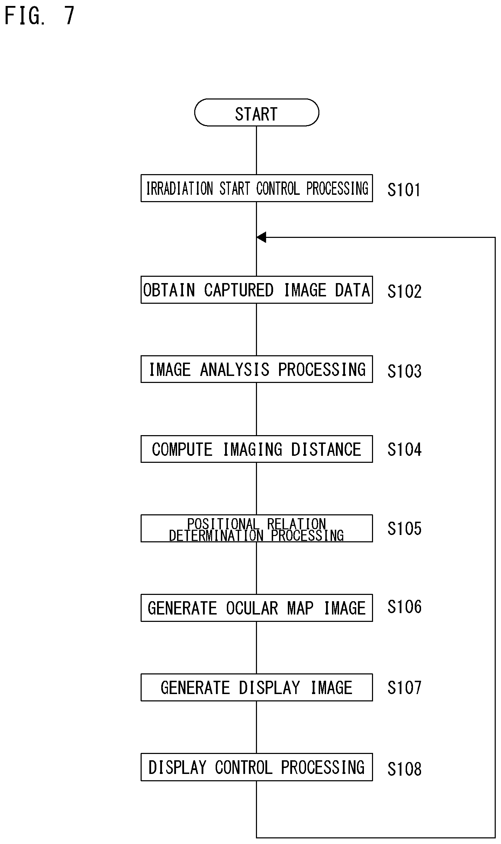

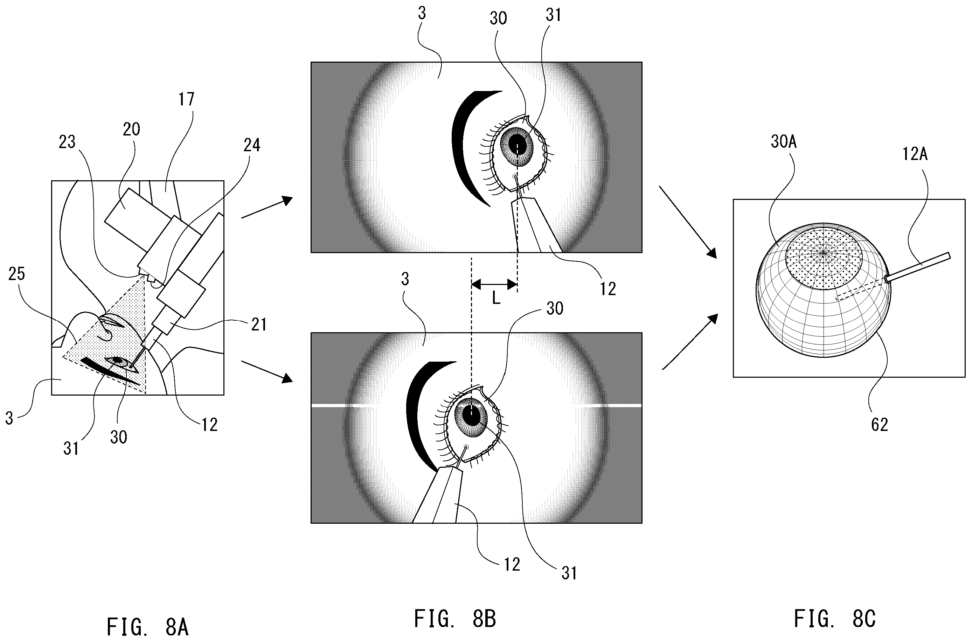

FIG. 7 is a flowchart illustrating an example of processing performed by the computation and control unit 14. In addition, FIG. 8 illustrates an outline of a procedure for generating the ocular map image 62.

In step S101, the computation and control unit 14 performs irradiation start control processing.

In the irradiation start control processing, the computation and control unit 14 causes the irradiating unit 23 to output light 25 for irradiating the eyeball 30 as illustrated in FIG. 8A. Incidentally, in FIG. 8A, the light 25 output from the irradiating unit 23 is schematically indicated by a broken line.

Thereafter, the computation and control unit 14 repeatedly performs the processing from step S102 on down in timing of each frame of the image.

In step S102, the computation and control unit 14 stores, in the internal memory, respective pieces of frame image data as the captured image data obtained by imaging the eyeball 30 by the imaging units 24L and 24R.

In step S103, on the basis of two pieces of captured image data as each frame, the computation and control unit 14 performs various kinds of image analysis processing such, for example, as recognition of light spots of the light 25 from the irradiating unit 23, which appear on the eyeball 30 or the cornea 31 of the eyeball 30.

In step S104, for the pair of captured image data (stereo images) obtained by the imaging units 24L and 24R, the computation and control unit 14 computes the imaging distance, which is a distance from the imaging unit 24 to the cornea 31, by a principle of triangulation from an amount of offset L between the positions of the light spots appearing on the cornea 31, as illustrated in FIG. 8B.

The computation and control unit 14 determines positional relation between the imaging unit 24 and the eyeball 30 on the basis of the computed imaging distance.

At this time, data indicating the three-dimensional ocular model 30A as illustrated in FIG. 8C (ocular model data) is used as data on the size of the eyeball 30.

The ocular model data is, for example, three-dimensional data assuming the eyeball size of an ordinary human. The eyeball size of humans does not differ greatly, though there are slight individual differences. Thus, the standard eyeball size of a human is set in advance as ocular model data.

The computation and control unit 14 can compute (determine) the positional relation between the imaging unit 24 and the eyeball 30 by using the imaging distance and the ocular model data.

Incidentally, it is also possible to measure the eyeball size of the eyeball 30 of the subject 3 in advance before the surgery and set the ocular model data by reflecting a result of the measurement.

In the following step S105, the computation and control unit 14 determines positional relation between the endoscope 12 and the eyeball 30 on the basis of the positional relation between the imaging unit 24 and the eyeball 30.

The relative positional relation of the endoscope 12 to the imaging unit 24 is fixed by fixing the endoscope 12 to the holder 21 of the arm distal end section 20. Therefore, in a state in which the endoscope 12 is fixed to the holder 21, the position of the endoscope 12 is defined naturally according to the position of the imaging unit 24.

In addition, a shape of the endoscope 12 up to the endoscope distal end portion 120 in an axial direction of the endoscope 12 fixed to the holder 21 is known. Therefore, when information regarding the shape of the endoscope 12 is set in advance, the computation and control unit 14 can compute the position of the endoscope distal end portion 120 from the defined position of the endoscope 12.

Hence, the computation and control unit 14 can compute (determine) the positional relation of the endoscope 12 (endoscope distal end portion 120) to the eyeball 30 on the basis of the positional relation between the eyeball 30 and the imaging unit 24.

In the following step S106, the computation and control unit 14 generates image data of the ocular map image 62 indicating the determined positional relation between the eyeball 30 and the endoscope 12 (endoscope distal end portion 120) as positional relation between the three-dimensional ocular model 30A and the endoscope model 12A.

In step S107, the computation and control unit 14 generates image data of the display image 6.

The computation and control unit 14 generates the image data of the display image 6 by synthesizing the endoscope captured image 61, the ocular map image 62, the endoscope viewpoint map image 63, the insertion length presenting image 64, and other necessary images.

Here, description will be made of an example of a method for generating image data of various kinds of images other than the ocular map image 62 described above.

### - Endoscope Captured Image 61

The computation and control unit 14 generates the image data of the endoscope captured image 61 on the basis of the captured image data captured by the endoscope 12.

### - Endoscope Viewpoint Map Image 63

The computation and control unit 14 generates image data of the endoscope viewpoint map image 63 displaying a three-dimensional model image of the subject 3 from the viewpoint of the endoscope 12.

Preset three-dimensional model data of a head portion of a human is used as the three-dimensional model of the subject 3. A value indicating the angle of the head portion of the subject 3 with respect to the endoscope 12 is set as head portion angle data in advance on an assumption that the subject 3 is laid down on his or her back on the operating table 1 and an installation angle of the endoscope holding device 11 with respect to the operating table 1 is defined.

The computation and control unit 14 generates the three-dimensional model image including the three-dimensional ocular model image on the basis of the three-dimensional model data and the head portion angle data.

Then, the computation and control unit 14 generates the image data of the endoscope viewpoint map image 63 by synthesizing the three-dimensional model image and the endoscope model image on the basis of the positional relation between the eyeball 30 and the endoscope 12.

### - Insertion Length Presenting Image 64

The computation and control unit 14 computes information regarding the insertion length of the endoscope 12 in the eyeball 30 and information regarding the distance from the endoscope distal end portion 120 to the retina 36 of the subject 3 from the positional relation of the endoscope 12 (endoscope distal end portion 120) with respect to the ocular model data determined in step S105.

Then, the computation and control unit 14 generates the image data of the insertion length presenting image 64 on the basis of these pieces of information.

The image data of the various kinds of images necessary to generate the image data of the display image 6 is generated by the method described above.

In the following step S108, the computation and control unit 14 performs display control for displaying the display image 6 on the liquid crystal of the monitor 15. The display image 6 as illustrated in FIG. 5 is thereby displayed within the same screen of the monitor 15.

Incidentally, the computation and control unit 14 may display, on another monitor 15, a part of the endoscope captured image 61, the ocular map image 62, the endoscope viewpoint map image 63, the insertion length presenting image 64, and other necessary images constituting the display image 6.

When the computation and control unit 14 completes the processing of step S108, the computation and control unit 14 returns the processing to step S102, and thereafter performs similar processing. By repeatedly performing the processing of steps S102 to S108, the computation and control unit 14 can update the ocular map image 62 when the position of the endoscope 12 is shifted.

### <4. Summary>

A surgery assistance device 2 in an embodiment of the present disclosure includes an arm unit 17 having a holder 21 for holding an endoscope 12, and configured to adjust a position of the endoscope 12 in a state in which the holder 21 holds the endoscope 12, a measuring unit 22 having fixed relative positional relation to the endoscope 12 held by the holder 21, and used to measure a distance to a surgery target region of a subject 3 (distance to the cornea 31 of the subject 3), a position determining section 143 configured to obtain positional relation between the surgery target region (eyeball 30) of the subject 3 and an endoscope distal end portion 120 on the basis of information from the measuring unit 22, an image generating section 144 configured to generate a map image (ocular map image 62) indicating the position of the endoscope 12 (endoscope model 12A) on a three-dimensional model (three-dimensional ocular model 30A) of the surgery target region (eyeball 30) by using a determination result of the position determining section 143, and a display control section 145 configured to perform display control of the map image (ocular map image 62) (see FIG. 6, FIG. 7, and the like).

Here, the surgery target region is a region of the subject 3 to be operated by an operator 4. According to the example of the present embodiment, the surgery target region is the eyeball 30 of the subject 3.

Consequently, the ocular map image 62 is displayed on a monitor 15. By checking the monitor 15, the operator 4 can recognize the position of the endoscope 12 inserted in the eyeball 30 of the subject 3.

Hence, the operator 4 can perform surgery while recognizing the position of the endoscope 12 inserted in the eyeball 30 of the subject 3. Hence, according to the present disclosure, it is possible to proceed with the surgery on the eyeball 30 smoothly. In addition, the operator 4 can perform the surgery while being aware of the distance of the endoscope distal end portion 120 to the retina 36. The safety of the surgery can therefore be improved.

In the surgery assistance device 2 in the present embodiment, the measuring unit 22 includes an irradiating unit 23 and an imaging unit 24 having fixed relative positional relation to the endoscope 12, and the position determining section 143 determines the positional relation between the eyeball 30 of the subject 3 and the endoscope distal end portion 120 on the basis of a captured image of a light spot of light 25 from the irradiating unit 23, the light spot appearing on the cornea 31 of the subject 3, the captured image being captured by the imaging unit 24 (see S102 to S105 in FIG. 7, FIG. 8, and the like).

A distance from the imaging unit 24 to the cornea 31 can be calculated on the basis of the captured image, for example. The positional relation between the eyeball 30 and the endoscope distal end 120 can be determined on the basis of the distance.

Hence, it is possible to determine the positional relation between the eyeball 30 and the endoscope distal end portion 120, the positional relation reflecting the position of the subject 3. Consequently, accuracy of determination of the positional relation of the endoscope 12 in the eyeball 30 can be improved, and the operator 4 can recognize the positional relation of the endoscope 12 in the eyeball 30 more accurately.

In the surgery assistance device 2 in the present embodiment, the display control section 145 displays the ocular map image 62 and a captured image captured by the endoscope 12 (endoscope captured image 61) within the same screen (see S108 in FIG. 7 and the like).

Because the ocular map image 62 and the endoscope captured image 61 are displayed within the same screen, when the operator 4 performs the surgery while checking the captured image of the inside of the eyeball 30, the operator 4 easily recognizes the positional relation of the endoscope 12 in the eyeball 30 at the same time. Hence, it is possible to proceed with the surgery on the eyeball 30 more smoothly.

In the surgery assistance device 2 in the present embodiment, the image generating section 144 updates the ocular map image 62 according to a shift in the position of the endoscope 12 (see S108 and S102 in FIG. 7 and the like).

Consequently, the ocular map image 62 indicating the most recent positional relation between the eyeball 30 and the endoscope 12 is displayed on the monitor 15.

Hence, the operator 4 can perform the surgery while recognizing the latest positional relation between the eyeball 30 and the endoscope 12, and can therefore proceed with the surgery on the eyeball 30 more smoothly.

In the surgery assistance device 2 in the present embodiment, the image generating section 144 generates a display image 6 including information regarding an insertion length of the endoscope 12 in the eyeball 30 and information regarding a distance from the endoscope distal end portion 120 to the retina 36 of the subject 3 (see S107 in FIG. 7 and the like).

Consequently, the numerical value of the insertion length of the endoscope 12 in the eyeball 30 and the numerical value of the distance to the retina 36 of the subject 3, for example, are displayed on the monitor 15.

Hence, in addition to visually recognizing the positional relation between the eyeball 30 and the endoscope 12 on the basis of the ocular map image 62, the operator 4 can recognize the positional relation also on the basis of the concrete numerical values.

It is to be noted that, while an example of an intraocular endoscope has been described as an example of the endoscope 12 in the present embodiment, the endoscope 12 is not limited to the intraocular endoscope. For example, it is possible to apply various endoscopes such as a thoracoscope inserted after an incision between ribs of the subject 3 and a laparoscope inserted after an incision in an abdomen.

In a case of a thoracoscope, for example, the surgery target portion is a chest portion of the subject 3, and the distance from the measuring unit 22 to the surgery target portion is a distance from the measuring unit 22 to a skin of a rib part of the subject 3. In addition, in a case of a laparoscope, the surgery target portion is various kinds of organs such as a liver of the subject 3, and the distance from the measuring unit 22 to the surgery target portion is a distance from the measuring unit 22 to a skin of an abdominal region of the subject 3.

Finally, the embodiment described in the present disclosure is merely illustrative, and the present disclosure is not limited to the foregoing embodiment. In addition, all of combinations of the configurations described in the embodiment are not necessarily essential to solving the problems. Further, the effects described in the present disclosure are merely illustrative, and are not limited. Other effects may be produced, or a part of the effects described in the present disclosure may be produced.

### Reference Signs List

2: Surgery assistance device
3: Subject
12: Endoscope
12A: Endoscope model
17: Arm unit
21: Holder
22: Measuring unit
23: Irradiating unit
24: Imaging unit
30: Eyeball
30A: Three-dimensional ocular model
31: Cornea
36: Retina
61: Endoscope captured image
62: Ocular map image
120: Endoscope distal end portion
143: Position determining section
144: Image generating section
145: Display control section

## Claims

1. A surgery assistance device (2) comprising:
an arm unit (17) having a holder (21) for holding an endoscope (12), and configured to adjust a position of the endoscope (12) in a state in which the holder (21) holds the endoscope (12);
a measuring unit (22) used to measure a distance to a surgery target region of a subject (3);
a position determining section (143) configured to obtain positional relation between the surgery target region of the subject (3) and the endoscope distal end portion (120) on a basis of information from the measuring unit (22);
an image generating section (144) configured to generate a map image indicating the position of the endoscope (12) on a three-dimensional model of the surgery target region by using a determination result of the position determining section (143); and
a display control section (145) configured to perform display control of the map image,
wherein
the surgery target region is an eyeball (30),
the measuring unit (22) includes an imaging unit (24) and an irradiating unit (23), and
the position determining section (143) is configured to determine a positional relation between the eyeball (30) of the subject (3) and the endoscope distal end portion (120) on a basis of a captured image of a light spot of light from the irradiating unit (23), the light spot appearing on the eyeball of the subject (3), the captured image being captured by the imaging unit (24),
**characterized in that** the measuring unit has a fixed relative positional relation to the endoscope held by the holder.

2. The surgery assistance device (2) according to claim 1, wherein
the display control section (145) displays the map image and a captured image captured by the endoscope (12) within a same screen.

3. The surgery assistance device (2) according to any one of claims 1 or 2, wherein
the image generating section (144) updates the map image according to a shift in the position of the endoscope (12).

## Patentansprüche

1. Chirurgie-Assistenzvorrichtung (2), enthaltend:
eine Armeinheit (17) mit einem Halter (21) zum Halten eines Endoskops (12) und konfiguriert, eine Position des Endoskop (12) in einem Zustand einzustellen, in dem der Halter (21) das Endoskop hält (12);
eine Messeinheit (22), die verwendet wird, eine Distanz zu einem Chirurgie-Zielbereich eines Probanden (3) zu messen;
einen Positionsbestimmungsteil (143), der konfiguriert ist, eine Positionsbeziehung zwischen dem Chirurgie-Zielbereich des Probanden (3) und dem distalen Endoskop-Endabschnitt (120) auf Basis von Informationen der Messeinheit (22) zu erhalten;
einen Bilderzeugungsteil (144), der konfiguriert ist, ein Kartenbild zu erzeugen, das die Position des Endoskops (12) auf einem dreidimensionalen Modell des Chirurgie-Zielbereichs unter Verwendung eines Bestimmungsergebnisses des Positionsbestimmungsteils (143) anzeigt; und
einen Anzeige-Steuerungsteil (145), der konfiguriert ist, eine Anzeige-Steuerung des Kartenbildes auszuführen,
wobei
der Chirurgie-Zielbereich ein Augapfel (30) ist,
die Messeinheit (22) eine Bildgebungseinheit (24) und eine Bestrahlungseinheit (23) einschließt, und
der Positionsbestimmungsteil (143) konfiguriert ist, eine Positionsbeziehung zwischen dem Augapfel (30) des Probanden (3) und dem distalen Endoskop-Endabschnitt (120) auf Basis eines aufgenommenen Bildes eines Lichtpunkts aus der Bestrahlungseinheit (23) zu bestimmen, wobei der Lichtpunkt auf dem Augapfel des Probanden (3) erscheint, wobei das aufgenommene Bild von der Bildgebungseinheit (24) aufgenommen wird, **dadurch gekennzeichnet, dass** die Messeinheit eine feststehende relative Positionsbeziehung zu dem vom Halter gehaltenen Endoskop aufweist.

2. Chirurgie-Assistenzvorrichtung (2) gemäß Anspruch 1, wobei
der Anzeige-Steuerungsteil (145) das Kartenbild und ein vom Endoskop (12) aufgenommenes Bild auf demselben Bildschirm anzeigt.

3. Chirurgie-Assistenzvorrichtung (2) gemäß einem der Ansprüche 1 oder 2, wobei
der Bilderzeugungsteil (144) das Kartenbild entsprechend einer Verschiebung der Position des Endoskops (12) aktualisiert.

## Revendications

1. Un dispositif d'assistance chirurgicale (2) comprenant :
une unité de bras (17) ayant un support (21) pour supporter un endoscope (12), et configurée pour ajuster la position de l'endoscope (12) dans un état où le support (21) supporte l'endoscope (12) ;
une unité de mesure (22) utilisée pour mesurer une distance à une région cible chirurgicale d'un sujet (3) ;
une section de détermination de position (143) configurée pour obtenir une relation positionnelle entre la région cible chirurgicale du sujet (3) et la portion distale de l'endoscope (120) sur la base des informations de l'unité de mesure (22) ;
une section de génération d'image (144) configurée pour générer une image cartographique indiquant la position de l'endoscope (12) sur un modèle tridimensionnel de la région cible chirurgicale en utilisant un résultat de détermination de la section de détermination de position (143) ; et
une section de contrôle d'affichage (145) configurée pour effectuer le contrôle d'affichage de l'image cartographique,
dans lequel
la région cible chirurgicale est un globe oculaire (30),
l'unité de mesure (22) comprend une unité d'imagerie (24) et une unité d'irradiation (23),
et
la section de détermination de position (143) est configurée pour déterminer une relation positionnelle entre le globe oculaire (30) du sujet (3) et la portion distale de l'endoscope (120) sur la base d'une image capturée d'un point lumineux provenant de l'unité irradiante (23), le point lumineux apparaissant sur le globe oculaire du sujet (3), l'image capturée étant capturée par l'unité d'imagerie (24), **caractérisé en ce que** l'unité de mesure a une relation positionnelle relative fixe avec l'endoscope (12) supporté par le support (21),.

2. Le dispositif d'assistance chirurgicale (2) selon la revendication 1, dans lequel
la section de contrôle d'affichage (145) affiche l'image cartographique et une image capturée par l'endoscope (12) sur un même écran.

3. Le dispositif d'assistance chirurgicale (2) selon l'une des revendications 1 ou 2, dans lequel
la section de génération d'image (144) met à jour l'image cartographique selon un changement de la position de l'endoscope (12).
